# EUROPEAN PATENT SPECIFICATION

(11) **EP 3 254 571 B1**
(45) Date of publication and mention of the grant of the patent: **04.11.2020**
(21) Application number: 16183160.7
(22) Date of filing: 08.08.2016
(51) Int. Cl.: A24F 47/00, A61M 15/06, A61M 11/04, A61M 15/00

(54) **UPPER-ASSEMBLY ELECTRONIC CIGARETTE ATOMIZER, FULLY ENCLOSED ELECTRONIC CIGARETTE HAVING THE UPPER-ASSEMBLY ATOMIZER AND METHODS OF ASSEMBLY THEREOF**
ZERSTÄUBER IM OBEREN BEREICH EINER ELEKTRONISCHEN ZIGARETTE, VOLLSTÄNDIG UMSCHLOSSENE ELEKTRONISCHE ZIGARETTE MIT DEM ZERSTÄUBER IM OBEREN BEREICH UND VERFAHREN ZUR ANORDNUNG DAVON
ATOMISEUR DE CIGARETTE ÉLECTRONIQUE À ENSEMBLE SUPÉRIEUR, CIGARETTE ÉLECTRONIQUE TOTALEMENT FERMÉE PRÉSENTANT L'ATOMISEUR DE CIGARETTE À ENSEMBLE SUPÉRIEUR ET PROCÉDÉS D'ASSEMBLAGE ASSOCIÉS

(30) Priority: 07.06.2016 CN 201620545025 U
(43) Date of publication of application: 13.12.2017
(73) Proprietor: Shenzhen Innokin Technology Co., Ltd., Shenzhen Guangdong 518104 (CN)
(72) Inventor: LI, Jian Wei, Shenzhen, Guangdong 518104 (CN)
(74) Representative: Hirsch & Partners

(56) References cited:
- WO-A1-2014/161181
- WO-A1-2015/149311
- WO-A1-2016/008217
- WO-A1-2016/049824
- CN-U- 203 137 031
- US-A1- 2015 189 918
- US-A1- 2016 100 633

## Description

The present invention relates to a daily necessity accessory, in particular to an upper-assembly, open-injection, and fully enclosed electronic cigarette atomizer, to a fully enclosed electronic cigarette having the upper-assembly atomizer and to assembly methods of the same.

### Background Art

An existing electronic cigarette product generally comprises a control box formed by a battery and a control circuit board, which is then connected to other components including an atomizer and a cigarette holder (Drip-Tip or Mouthpiece). The structure of an electronic cigarette atomizer is generally as follows: a circular atomizing core and a connecting pipe are arranged in the central position in a circular enclosed space, and the remaining annular space is used to hold an e-liquid (cigarette liquid). The atomizing core and the connecting pipe are also closed; only two symmetrical liquid inlet holes are arranged on the outer wall of the atomizing core to supply the e-liquid to the atomizing core. By the connection to the control box, the atomizing core heats so that the e-liquid that enters the atomization core is atomized and aspirated through the connecting pipe.

An electronic cigarette with such a structure generally comprises two parts: an atomizer and a battery box. In order to allow smooth connection between the atomization core and the battery box, the atomizer and the battery box need to be respectively provided with a special connection mechanism. For example, an external thread conductive column is arranged at the bottom of the atomizer, and an internal collection hole is arranged in the battery box. The function of the electronic cigarette can be realized only if the conductive column and the battery box are electrically connected through a threaded connection. Therefore, an atomizer is generally powered on at the bottom. For power-on at the bottom, atomization and heating components, including the atomization core of the atomizer, need to be located at the bottom of the atomizer and fixed at the bottom of the atomizer. With the atomization core arranged at the bottom, an air inlet used for introducing fresh air is also arranged at the bottom. As the air inlet communicates with the atmosphere, the e-liquid in the atomizer is prone to leak through the air inlet, leading to pollution and waste. In addition, due to a structure with lower air intake and lower power-on, it is difficult to replace the atomization core. During e-liquid injection, attention must be paid to the problem of liquid leakage through the lower air inlet hole.

However, as long as the atomizer is powered on at the bottom, the atomization core has to be assembled in the lower part; as long as air intake is through the lower part, liquid leakage through the air inlet cannot be avoided. In addition, such a structure with lower air intake and lower power-on is complicated; therefore, the assembly is difficult.

In order to overcome the aforesaid defects, the inventor has designed a new type of electronic cigarette atomizer, which can achieve the effects of upper power-on, upper air intake, and full closure.

The following document also disclose electronic cigarettes with different atomizer assemblies, US2016/0100633, WO2016/049824, CN203137031U, WO2014/161181, US2015/0189918, WO2016/008217, WO2015/149311.

### Summary of the Invention

The invention is defined in the appended claims. The purpose of the invention is to provide an upper-assembly electronic cigarette atomizer and a method of assembly of the same, in which, by a change in the atomizer structure, the atomization core is powered on in the upper part; air is taken in from the upper part; and an e-liquid (cigarette liquid) is injected through an opening on the top of the atomizer, allowing convenient liquid injection and full closure to prevent liquid leakage.

An upper-assembly electronic cigarette atomizer according to the present invention comprises respectively, from bottom up, a liquid storage cup having an upper opening and a head cover component connected with the upper opening of the liquid storage cup in a closed and threaded manner to form a closed space in the liquid storage cup for containing an e-liquid (cigarette liquid), and the head cover component is an integrated part which is configured to be removed together from the liquid storage cup. The head cover component according to the present invention further comprises a cigarette holder (Drip-Tip or Mouthpiece), a liquid storage cup cover, a connecting electrode, an aspiration pipe, an air intake pipe, and an atomization device, with the aspiration pipe, the air intake pipe, and the atomization device extended downward into the liquid storage cup.

The aforesaid liquid storage cup cover has a multilayer structure; from bottom up, the layers are respectively the liquid storage cup cover body serving also as a first electrode, an insulating ring, an air intake ring chamber serving also as a second electrode, and an air intake regulating ring, wherein the liquid storage cup cover body is annularly and electrically connected with the air intake pipe; the air intake ring chamber cover and the aspiration pipe are annularly and electrically connected; the air intake regulating ring is arranged in the periphery of the air intake ring chamber for rotary joint.

The aforesaid atomization device comprises an atomizing core body and an outer shell, which are preferably both cylindrical, the atomizing core body being fixedly arranged in the outer shell. The upper end of the atomizing core body is annularly connected with the lower end of the aspiration pipe in an insulating manner, with the lower end opened. The upper end of the outer shell is annularly and electrically connected with the lower end of the air intake pipe, with the lower end closed. The atomizing core body is provided with an electric heating device and a radial liquid inlet; the outer shell is provided with a radial inlet hole; the position of the liquid inlet of the outer shell corresponds to the position of the liquid inlet of the atomizing core body. The outer edge of the atomizing core body comes into contact with the inner wall of the outer shell for connection, and a ventilation notch is arranged between the outer edge of the atomizing core body and the outer shell. Two terminals of the electric heating device of said atomizing core body are electrically connected with the atomizing core body and the aspiration pipe respectively.

A ventilation notch between said atomizing core body and the outer shell is arranged on the outer wall of the atomizing core body; the outer wall is provided with an axial notch that is depressed towards the axis; the notched part comes into contact with the outer shell to form a ventilation notch.

The outer shell of said atomization device comprises a cylindrical portion and a cup-shaped portion, the cylindrical portion and a cup-shaped portion being interconnected in a closed manner; said atomizing core body is fixedly arranged in the outer shell of the atomization device; the cylindrical upper end of the outer shell is annularly connected to the air intake pipe in a closed manner.

The cylindrical portion of said outer shell is sheathed on the outer layer of the atomizing core body; the cup-shaped portion and the lower part of the atomizing core body are interconnected in a threaded manner; the cylindrical portion of said outer shell and the air intake pipe are interconnected in a threaded manner.

A buffer insulating ring is arranged between said atomizing core body and the aspiration pipe; a buffer ventilating ring is arranged between the atomizing core body and the cup-shaped portion bottom of the outer shell; the upper section of said buffer ventilating ring is provided with an axial hole, and the lower section is provided with a radial hole; the axial hole communicates with the radial hole; the bottom of the buffer ventilating ring end is pressed tightly against the bottom of the outer shell.

An electronic cigarette comprising an upper-assembly electronic cigarette atomizer according to the present invention is also provided.

A method of assembly of the atomizer according to the present invention comprises an assembly of the atomization device and an upper part of the head cover component separately and a connection of the assembled atomization device to a lower end of the air intake pipe from a lower end of the head cover component.

A method of assembly of the electronic cigarette according to the present invention comprising an injection of the e-liquid into the liquid storage cup to form a cartridge; a montage of the head cover component on the liquid storage cup containing e-liquid; and a montage of the upper-assembly electronic cigarette atomizer on the electronic cigarette.

In the present invention, the atomization device, air intake pipe, aspiration pipe, and connecting electrodes are all assembled into the head cover assembly. After the head cover component is connected to the upper opening (cup mouth portion) of the liquid storage cup, the atomization device, aspiration pipe, and air intake pipe extend into the e-liquid in the liquid storage cup. After the connecting electrode arranged in the head cover component is connected to the power supply, the effect of power-on in the upper part can be achieved. With power-on in the upper part, a structure with power-on in the lower part, air intake in the lower part, and assembly at both the upper and lower ends is completely avoided. Such a structure is simple and easy to assemble; in addition, as the cartridge is completely closed, the liquid leakage problem with the lower assembly is eliminated.

### Brief Description of the Drawings

Figure 1 shows a sectional structural diagram for an upper-assembly electronic cigarette atomizer according to an embodiment of the present invention;
Figure 2 shows a sectional structural diagram for an upper-assembly electronic cigarette atomizer in an embodiment of the present invention axially rotated by an angle of 90 degrees;
Figure 3 shows a three-dimensional breakdown structural diagram for an atomization device in the present invention;
Figure 4 shows a three-dimensional structural diagram for a ventilating ring in the present invention;
Figure 5 shows an abbreviated drawing for the use state of an atomizer in an embodiment of the present invention;
Figure 6 shows a three-dimensional structural diagram for the assembly of an embodiment of the present invention.

Symbols used in the drawings: 1. Cigarette holder (Drip-Tip or Mouthpiece); 2. Connecting pipe of the aspiration pipe; 3. Air intake annular chamber; 4. Air inlet on the side wall; 5. Aspiration pipe; 6. Air intake pipe; 7. Connection thread; 8. Cylindrical portion of the outer shell; 9. Atomizing core body; 10. Insulating ventilating ring group; 11. Air intake regulating ring; 12. Thread electrode; 13. Insulating ring; 14. Liquid storage cup cover body; 15. Liquid storage cup; 16. Buffer insulating ring group; 17. Liquid inlet of the atomizing core body; 18. Cup-shaped portion of the outer shell; 19. Air intake regulating hole; 20. Liquid inlet of the outer shell; 21. Atomization airway; 22. Ventilation notch of the atomizing core body; 23. Axial ventilating hole of the ventilating ring; 24. Radial ventilating hole of the ventilating ring; 25. Upper current electrode of the electronic cigarette; 26 Lower current electrode of the electronic cigarette.

### Detailed Description of the Invention

A further detailed description of the invention is provided below with the help of attached drawings and embodiments. However, the drawings and embodiments are only intended to explain the present invention, instead of limiting the protection scope of the present invention.

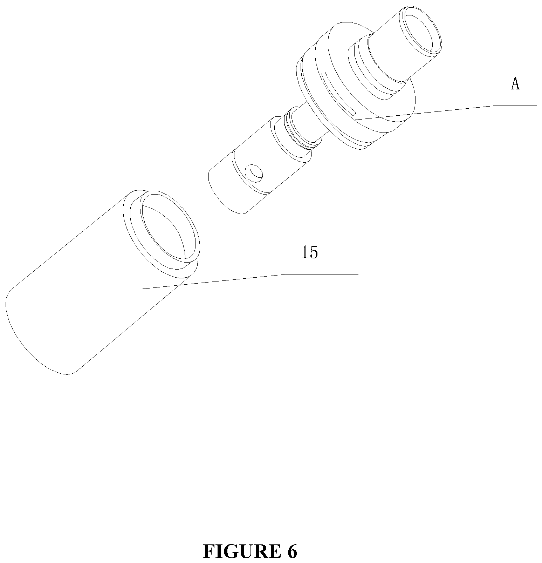

As shown in Figure 6, the electronic cigarette atomizer of this embodiment comprises respectively, from top to bottom, a head cover component A and a liquid storage cup 15 having an upper opening (cup mouth portion); the head cover component A and the liquid storage cup 15 are interconnected at the upper opening (cup mouth portion) in a closed and threaded manner. Thus, in the liquid storage cup 15 is formed a closed space; the closed space is used for injecting an e-liquid to form a cartridge. As such a cartridge is completely closed, no liquid leakage will occur.

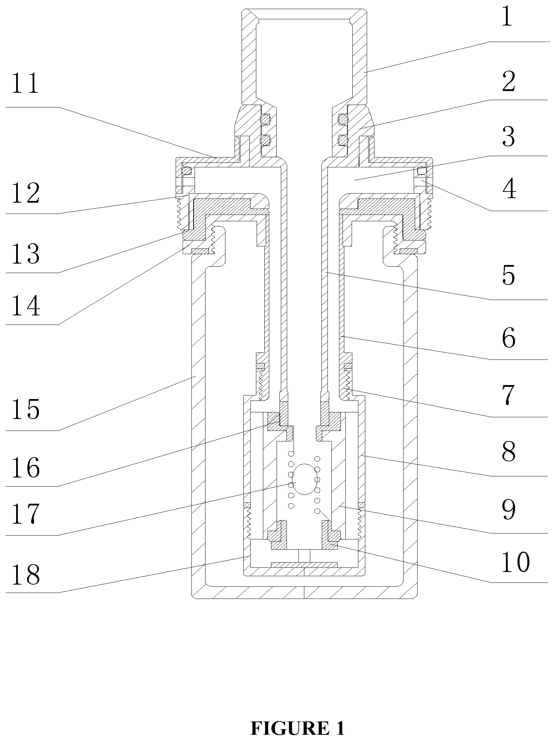

As shown in Figure 1 and Figure 6, in the electronic cigarette atomizer of this embodiment, other than the liquid storage cup 15, all the other components form an integrated head cover component A. The head cover component A further comprises a cigarette holder (Drip-tip or Mouthpiece) 1, a liquid storage cup cover, connecting electrodes, an aspiration pipe 5, an air intake pipe 6, and an atomization device. The atomization device, the aspiration pipe 5, and the air intake pipe 6 extend into the liquid storage cup and remain in the e-liquid during use. When the head cover component is removed from the liquid storage cup 15, the whole of the aforesaid device can be removed together. An e-liquid can be easily replaced and injected.

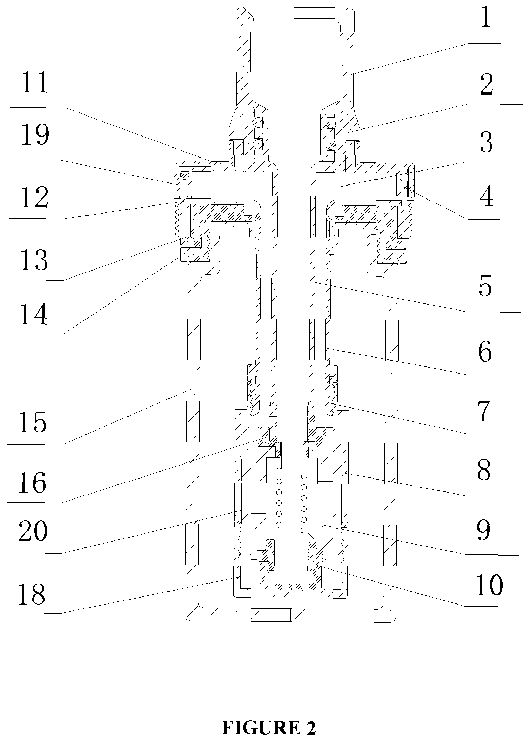

As shown in Figure 1 and Figure 2, the atomization device of the embodiment comprises an atomizing core body 9 and an outer shell, the outer shell further comprising a cylindrical portion 8 and a cup-shaped portion 18. The cup-shaped portion 18 is tightly connected with the cylindrical portion 8 to form an integral outer shell with a closed bottom. The upper end of the cylindrical portion 8 of the outer shell is connected with an air intake pipe 6 by a screw thread 7 in a threaded and sealed manner so that the outer shell of the atomization device is connected with the air intake pipe to form the head cover component A.

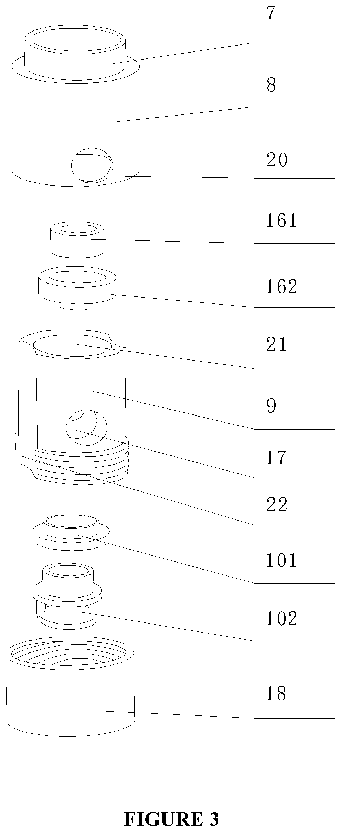

As shown in Figure 1 and Figure 2, the atomizing core body 9 of the atomization device of this embodiment is arranged in the outer shell. As shown in Figure 3, the atomizing core body 9 is a cylinder with its body having a circular cross-section. The cylinder is provided with an axial atomization airway 21. In the axial atomization airway 21, an electric heating device is arranged, for example, a resistance wire heater and a liquid-guiding cotton (not marked in figures). In addition, a radial liquid inlet 17 is arranged on the cylinder wall of the atomizing core body 9; the liquid inlet 17 is used for guiding an e-liquid.

As shown in Figure 1 and Figure 2, the atomizing core body 9 is assembled tightly pressed against the interior of the outer shell; the outer wall of the atomizing core body 9 is tightly pressed against the inner wall of the outer shell. Consequently, the channel for upper air intake is blocked in this setting mode. Therefore, the outer wall of the atomizing core body 9 is provided with an axial ventilation notch 22 depressed towards the axis. The axial ventilation notch 22 is used to introduce the air in the upper part of the atomizing core body 9 into the lower part of the body 9.

On the cylinder wall of the cylindrical portion 8 of the outer shell and in the atomization device, in the position corresponding to the liquid inlet 17 of the atomizing core body 9, an outer shell liquid inlet 20 is arranged to lead in an e-liquid.

See Figure 3. The closed connection between the cylindrical portion 8 of the outer shell in the atomization device and the cup-shaped portion 18 is implemented as follows: In the lower section of the atomizing core body 9, an external thread is arranged; on the cup-shaped portion 18 of the outer shell, an internal thread is arranged. Through the threads, the atomizing core body 9 is connected to the cup-shaped portion 18. Through the connection, the cylindrical portion 8 outside the upper section of the atomizing core body 9 is connected to the cup-shaped portion 18. In order to achieve the sealing effect, a sealing ring is arranged at the joint (the sealing ring is not marked in the figure).

As shown in Figure 1 and Figure 2, in order to ensure that the installation position of the atomizing core body 9 in the outer shell is fixed, a stable and reliable electrical connection is established. In this embodiment, the upper side of the atomizing core body 9 is provided a buffer insulating ring group 16; the buffer insulating ring group 16 comprises a buffer pressure ring 161 and an insulating ring 162, as shown in Figure 3; both rings are made of insulating materials with certain compression space, such as silica gel. Its purpose is to ensure that the connection between the atomizing core body 9 and the aspiration pipe 5 is provided with a rigid buffering process, while achieving mutual insulation. The buffer insulating ring group 16 is pressed tightly against the bottom of the aspiration pipe 5. Thus, buffering is performed while the insulation effect is guaranteed.

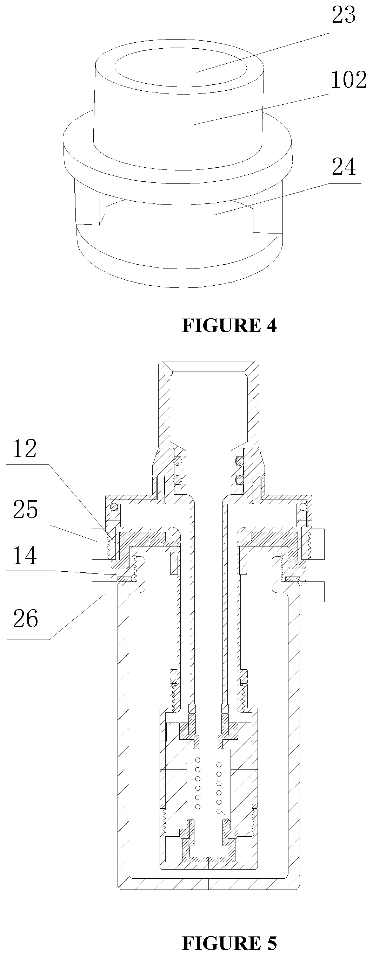

As shown in Figure 1, Figure 2, and Figure 3, the lower part of the atomizing core body 9 is provided with a buffer ventilating ring group 10. As shown in Figure 3, the buffer ventilating ring group 10 comprises the buffer ring 101 and the ventilating ring 102. The rings are both made of insulating materials with a certain compression capability, such as silica gel. As shown in Figure 4, the top of the ventilating ring 102 is provided with an axial ventilating hole 23 that communicates with the atomization airway 21 of the atomizing core main body 9; in addition, the lower end of the ventilating ring 102 is provided with a radial ventilating hole 24; the axial ventilating hole 23 and the radial ventilating hole 24 communicate with each other. When the atomizing core body 9 and the bottom of the cup-shaped 18 are arranged pressed against each other via the buffer ventilating ring group 10, the atomizing core body 9 can be fixed; in addition, fresh air can be supplied to the atomizing core body.

As shown in Figure 1 and Figure 2, the atomization device of the present invention is a combination body arranged at the lowermost end of the head cover component A; it is connected to a head portion of the head cover component A through the aspiration pipe 5 and the air intake pipe 6. The connection mode is implemented by connecting the outer shell cylindrical portion 8 to the lower end of the air intake pipe 6 through the screw thread 7. In addition, the buffer insulating ring group 16 in the upper part of the atomizing core body 9 is tightly pressed against the lower end of the aspiration pipe 5 for connection.

In the head cover component A, the liquid storage cup cover of the head cover component A has a multilayer structure, the bottom layer of the liquid storage cup cover is a liquid storage cup cover body 14; the liquid storage cup cover body 14 is an annular structure and serves as a first electrode; the first electrode is annularly and electrically connected with the upper end of the air intake pipe 6.

The next upper layer is an insulating ring 13. The upper layer of the insulating ring 13 is a double-layer annular chamber having an upper cover. The annular chamber is the air intake annular chamber 3 of this embodiment which serves also as a second electrode. The upper cover of the air intake annular(ring) chamber 3 is annually and electrically connected to the aspiration pipe 5 and serves as a second electrode.

The side wall of the air intake annular chamber 3 is provided with an air inlet 4, and an air intake regulating ring 11 is arranged on the outer layer of the air intake annular chamber 3; the air intake regulating ring 11 can rotate relative to the annular chamber; the air intake regulating ring 11 is provided with the corresponding air intake regulating hole 19; by rotating the air intake regulating ring 11, the opening size of the air inlet 4 can be adjusted to regulate the air flow rate.

The electrical connection structure of the present invention is as follows. As shown in Figure 5, the liquid storage cup cover body 14 is a conductor with an annular structure; the lower side of the annular outer edge is provided with a connection contact shoulder; the ventilating annular chamber 3 is a conductor, with its outer side wall provided with a connection crew thread 12. With such an arrangement, the whole atomizer can be connected to a battery box through the crew thread 12 in a threaded manner; devices including the battery box are further provided with a lower current electrode 26 used to be pressed tightly against the outer edge of the liquid storage cup 14 and an upper current electrode 25 that is connected to the crew thread 12, thus establishing an electrical connection.

The electric connection structure in the atomizer is as follows: the liquid storage cup cover body 14 is electrically connected to air intake pipe 6 and is further connected to the outer shell of the atomization device. Then, a first electrode is formed by connecting to the atomizing core body 9 through the outer shell of the atomization device. The screw thread 12, through the chamber wall of the ventilating annular chamber 3, is electrically connected to the upper cover of the annular chamber to form a second electrode, then is connected to the aspiration pipe 5, and is led into an electric heating device in the atomizing core body 9 through a lead arranged at the lower end of the aspiration pipe 5.

During the assembly of an atomizer according to this embodiment, the atomization device arranged at the lowermost end of the head cover component A and the upper part of the head cover component A are assembled separately. The purpose of dividing the outer shell of the atomization device into two parts is to facilitate the assembly.

First, assemble the atomization device. Install a buffer ventilating ring group 10 in the lower part of the atomizing core body 9 of the atomization device; then, from the lower part of the atomizing core body 9, install the outer shell cup-shaped portion 18; then, on the atomizing core body 9, install a sealing ring; then, from the upper section of the atomizing core body 9, install the cylindrical portion 8; then, install the buffer insulating ring group 16. Complete the assembly of the atomization device.

Then, install the upper part of the head cover component A. During the assembly, the aspiration pipe 5 is used as the benchmark. The upper end of the aspiration pipe 5 is provided with a connecting tube 2 used for mounting a cigarette holder (Drip-tip or Mouthpiece)1; the annular platform of the connecting pipe 2 is used as an installation locking platform. Therefore, it is necessary to first mount the air intake adjusting ring 11; then, install the top cover of the air intake annular chamber 3, side wall of the air intake annular chamber 3, and the bottom cover of the air intake annular chamber 3; under the bottom cover of the air intake annular chamber, further arrange an insulating ring 13; under the insulating ring 13, further arrange a liquid storage cup cover body 14; then, tightly fit the liquid storage cup cover body 14 with the air intake pipe for fixed connection. Then, fixedly connect the assembled atomization device to the lower end of the air intake pipe 6 from the lower end and with the screw thread 7.

To assemble and use the electronic cigarette with the upper-assembly electronic cigarette atomizer according to the present invention, firstly inject an e-liquid into the liquid storage cup 15; then, add the head cover component A; mount the whole atomizer on the electronic cigarette; switch on the power supply. The e-liquid in the liquid storage cup 15, through the outer shell liquid inlet 20 and the liquid inlet 17 of the atomizing core body, enters the atomization airway 21 of the atomizing core, is atomized in the atomization airway 21, and then is aspirated. Fresh air enters through the air inlet 4 on the side wall of the air intake annular chamber 3 at the upper end, and enters the atomization device through the annular space between the air intake pipe 6 and the aspiration pipe 5. In the atomization device, the fresh air enters the bottom of the air intake pipe through the ventilating notch 22 of the atomizing core body 9; through the buffer ventilating ring group 10, the fresh air enters the bottom of the atomizing core body 9 to be used by the atomization core.

With the atomizer according to the present invention, the cartridge is fully closed; thus, no e-liquid leaks from the cartridge; such a structure allowing upper assembly, upper power-on, and upper air intake is much easier in assemble. In addition, it makes e-liquid injection more convenient and safer.

## Claims

1. An upper-assembly electronic cigarette atomizer, wherein said atomizer comprises respectively, from bottom up:
- a liquid storage cup (15) having an upper opening; and
- a head cover component (A) connected with the upper opening of the liquid storage cup (15);
wherein the liquid storage cup (15) is connected to the head cover component (A) in a closed and threaded manner to form a closed space in the liquid storage cup (15) for containing an e-liquid;
wherein the head cover component (A) comprises a cigarette holder (1), a liquid storage cup cover, connecting electrodes, an aspiration pipe (5), an air intake pipe (6), and an atomization device, and is an integrated part configured to be removed together from the liquid storage cup (15), wherein the aspiration pipe (5), the air intake pipe (6), and the atomization device extended downward into the liquid storage cup (15) ;
wherein said liquid storage cup cover has a multilayer structure, from bottom up, the layers are respectively the liquid storage cup cover body (14) serving also as a first electrode (26), an insulating ring (13), an air intake ring chamber (3) having an upper cover and serving as a second electrode (25), and an air intake regulating ring (11), the liquid storage cup cover body is annularly and electrically connected with the air intake pipe; the air intake ring chamber cover and the aspiration pipe are annularly and electrically connected; the air intake regulating ring is arranged in the periphery of the air intake ring chamber for rotary joint.

2. The upper-assembly electronic cigarette atomizer according to claim 1, **characterized in that** said atomization device comprises an atomizing core body (9) and an outer shell, preferably both cylindrical.

3. The upper-assembly electronic cigarette atomizer according to claim 2, **characterized in that** the atomizing core body (9) being fixedly arranged in the outer shell; the upper end of the atomizing core body is annularly connected with the lower end of the aspiration pipe in an insulating manner, with the lower end opened; the upper end of the outer shell is annularly and electrically connected with the lower end of the air intake pipe (6), with the lower end closed; the atomizing core body is provided with an electric heating device and a radial liquid inlet (21); the outer shell is provided with a radial inlet hole (17); the position of the liquid inlet of the outer shell corresponds to the position of the liquid inlet of the atomizing core body; the outer edge of the atomizing core body comes into contact with the inner wall of the outer shell for connection, and a ventilation notch (22) is arranged between the outer edge of the atomizing core body and the outer shell; two terminals of the electric heating device of said atomizing core body are electrically connected with the atomizing core body and the aspiration pipe respectively.

4. The upper-assembly electronic cigarette atomizer according to claim 2 or 3, **characterized in that** a ventilation notch (22) between said atomizing core body and the outer shell is arranged on the outer wall of the atomizing core body; the outer wall is provided with an axial notch that is depressed towards the axis; the notched part comes into contact with the outer shell to form the ventilation notch (22).

5. The upper-assembly electronic cigarette atomizer according to anyone of claims 2 to 4, **characterized in that** the outer shell of said atomization device comprises a cylindrical portion (8) and a cup-shaped portion (18), the cylindrical portion (8) and the cup-shaped portion (18) being interconnected in a closed manner; said atomizing core body is fixedly arranged in the outer shell of the atomization device; the cylindrical upper end of the outer shell is annularly connected to the air intake pipe (6) in a closed manner.

6. The upper-assembly electronic cigarette atomizer according to claim 5, **characterized in that** the cylindrical portion of said outer shell is sheathed on the outer layer of the atomizing core body; the cup-shaped portion and the lower part of the atomizing core body are interconnected in a threaded manner; the cylindrical portion of said outer shell and the air intake pipe are interconnected in a threaded manner.

7. The upper-assembly electronic cigarette atomizer according to anyone of claims 2 to 6, **characterized in that** a buffer insulating ring (16) is arranged between said atomizing core body and the aspiration pipe; a buffer ventilating ring (10) is arranged between the atomizing core body and the cup-shaped portion bottom of the outer shell; the upper section of said buffer ventilating ring is provided with an axial hole, and the lower section is provided with a radial hole; the axial hole communicates with the radial hole; the bottom of the buffer ventilating ring end is pressed tightly against the bottom of the outer shell.

8. An electronic cigarette, **characterized in that** said electronic cigarette comprises an upper-assembly electronic cigarette atomizer according to anyone of claims 1 to 7.

9. A method of assembly of the atomizer according to anyone of claims 1 to 7, comprising:
- an assembly of the atomization device and an upper part of the head cover component separately and
- a connection of the assembled atomization device to a lower end of the air intake pipe from a lower end of the head cover component.

10. The method of assembly of an atomizer according to claim 9, wherein the assembly of the atomization device comprises:
- an installation of a buffer ventilating ring group (10) in the lower part of an atomizing core body (9) of the atomization device;
- an installation of, from the lower part of the atomizing core body (9), an outer shell cup-shaped portion (18);
- an installation of, on the atomizing core body (9), a sealing ring;
- an installation of, from the upper section of the atomizing core body, an outer shell cylindrical portion (8); and,
- an installation of a buffer insulating ring group (16).

11. The method of assembly of an atomizer according to claim 9 or 10, wherein the aspiration pipe is used as a benchmark and wherein the upper end of the aspiration pipe is provided with a connecting tube used for mounting a cigarette holder; wherein an annular platform of the connecting pipe is used as an installation locking platform; the assembly of the upper part of the head cover component comprises:
- a montage of an air intake adjusting ring;
- an installation of the top cover of the air intake annular chamber, side wall of the air intake annular chamber, and the bottom cover of the air intake annular chamber; under the bottom cover of the air intake annular chamber, and
- an arrangement of an insulating ring; under the insulating ring, and
- an arrangement of a cover body of liquid storage cup.

12. The method of assembly of the atomizer according to anyone of claim 9 to 10, further comprising a tight connection of the cover body of the liquid storage cup with the air intake pipe of the head cover component.

13. A method of assembly of the electronic cigarette according claim 8, comprising:
- an injection of the e-liquid into the liquid storage cup to form a cartridge;
- a montage of the head cover component on the liquid storage cup containing the e-liquid; and
- a montage of the upper-assembly electronic cigarette atomizer on the electronic cigarette.

## Patentansprüche

1. Ein oben montierter Zerstäuber für eine elektronische Zigarette, wobei der Zerstäuber von unten nach oben entsprechend umfasst:
- einen Flüssigkeitsbeinhaltungsbecher (15) mit einer oberen Öffnung; und
- eine Kopfabdeckungskomponente (A), die mit der oberen Öffnung des Flüssigkeitsbeinhaltungsbechers (15) verbunden ist; wobei der Flüssigkeitsbeinhaltungsbecher (15) mit der Kopfabdeckungskomponente (A) eng und gewindemäßig verbunden ist, um einen geschlossenen Raum in dem Flüssigkeitsbeinhaltungsbecher (15) zum Enthalten einer E-Flüssigkeit zu bilden;
.wobei die Kopfabdeckungskomponente (A) einen Zigarettenhalter (1), eine Flüssigkeitsbeinhaltungsbecher-Abdeckung, Verbindungselektroden, ein Ansaugrohr (5), ein Lufteinlassrohr (6) und eine Zerstäubungsvorrichtung umfasst und ein integrierter Teil ist, der eingerichtet ist, um zusammen von dem Flüssigkeitsbeinhaltungsbecher (15) entfernt zu werden, wobei sich das Ansaugrohr (5), das Lufteinlassrohr (6) und die Zerstäubungsvorrichtung nach unten in den Flüssigkeitsbeinhaltungsbecher (15) erstrecken
wobei die Flüssigkeitsbeinhaltungsbecher-Abdeckung eine mehrschichtige Struktur aufweist, wo von unten nach oben die Schichten entsprechend dem Flüssigkeitsbeinhaltungsbecher-Abdeckungskörper (14), welcher auch als eine erste Elektrode (26) dient, einem Isolierring (13), einer Lufteinlass-Ringkammer (3), die eine obere Abdeckung aufweist und als eine zweite Elektrode (25) dient, und einem Lufteinlass-Regulierungsring (11) entsprechen, wo der Flüssigkeitsbeinhaltungsbecher-Abdeckungskörper mit dem Lufteinlassrohr zu einem Ring und elektrisch verbunden ist; wobei die Lufteinlass-Ringkammerabdeckung und das Ansaugrohr zu einem Ring und elektrisch verbunden sind; wobei der Lufteinlass-Regulierungsring in dem Umfang der Lufteinlass-Ringkammer zur Drehverbindung angeordnet ist.

2. Der oben montierter Zerstäuber für eine elektronische Zigarette nach Anspruch 1, **dadurch gekennzeichnet, dass** die Zerstäubungsvorrichtung einen Zerstäubungskernkörper (9) und eine äußere Hülle umfasst, die beide vorzugsweise zylindrisch sind.

3. Der oben montierter Zerstäuber für eine elektronische Zigarette nach Anspruch 2, **dadurch gekennzeichnet, dass** der Zerstäubungskernkörper (9) in der äußeren Hülle fest angeordnet ist; das obere Ende des Zerstäubungskernkörpers mit dem unteren Ende des Ansaugrohrs in einer isolierenden Weise zu einem Ring verbunden ist, wobei das untere Ende offen ist; das obere Ende der äußeren Hülle mit dem unteren Ende des Lufteinlassrohrs (6) zu einem Ring und elektrisch verbunden ist, wobei das untere Ende geschlossen ist; der Zerstäubungskernkörper mit einer elektrischen Heizvorrichtung und einem radialen Flüssigkeitseinlass (21) vorgesehen ist; die äußere Hülle mit einem radialen Einlassloch (17) vorgesehen ist; die Position des Flüssigkeitseinlasses der äußeren Hülle der Position des Flüssigkeitseinlasses des Zerstäubungskernkörpers entspricht; der äußere Rand des Zerstäubungskernkörpers mit der inneren Wand der äußeren Hülle zur Verbindung in Kontakt kommt und eine Entlüftungskerbe (22) zwischen dem äußeren Rand des Zerstäubungskernkörpers und der äußeren Hülle angeordnet ist; zwei Anschlüsse der elektrischen Heizvorrichtung des Zerstäubungskernkörpers mit dem Zerstäubungskernkörper bzw. dem Ansaugrohr elektrisch verbunden sind.

4. Der oben montierter Zerstäuber für eine elektronische Zigarette nach Anspruch 2 oder 3, **dadurch gekennzeichnet, dass** eine Entlüftungskerbe (22) zwischen dem Zerstäubungskernkörper und der äußeren Hülle auf der äußeren Wand des Zerstäubungskernkörpers angeordnet ist; die äußere Wand mit einer axialen Kerbe vorgesehen ist, die hin zu der Achse vertieft ist; der eingekerbte Teil mit der äußeren Hülle in Kontakt kommt, um die Entlüftungskerbe (22) zu bilden.

5. Der oben montierter Zerstäuber für eine elektronische Zigarette nach einem der Ansprüche 2 bis 4, **dadurch gekennzeichnet, dass** die äußere Hülle der Zerstäubungsvorrichtung einen zylindrischen Abschnitt (8) und einen becherförmigen Abschnitt (18) umfasst, wobei der zylindrische Abschnitt (8) und der becherförmige Abschnitt (18) in enger Weise miteinander verbunden sind; der Zerstäubungskernkörper in der äußeren Hülle der Zerstäubungsvorrichtung fest angeordnet ist; das zylindrische obere Ende der äußeren Hülle mit dem Lufteinlassrohr (6) in enger Weise zu einem Ring verbunden ist.

6. Der oben montierter Zerstäuber für eine elektronische Zigarette nach Anspruch 5, **dadurch gekennzeichnet, dass** der zylindrische Abschnitt der äußeren Hülle die äußere Schicht des Zerstäubungskernkörpers ummantelt; der becherförmige Abschnitt und der untere Teil des Zerstäubungskernkörpers gewindemäßig miteinander verbunden sind; der zylindrische Abschnitt der äußeren Hülle und das Lufteinlassrohr gewindemäßig miteinander verbunden sind.

7. Der oben montierter Zerstäuber für eine elektronische Zigarette nach einem der Ansprüche 2 bis 6, **dadurch gekennzeichnet, dass** ein Pufferisolierring (16) zwischen dem Zerstäubungskernkörper und dem Ansaugrohr angeordnet ist; ein Pufferentlüftungsring (10) zwischen dem Zerstäubungskernkörper und dem becherförmigen Abschnittboden der äußeren Hülle angeordnet ist; der obere Abschnitt des Pufferentlüftungsrings mit einem axialen Loch vorgesehen ist und der untere Abschnitt mit einem radialen Loch vorgesehen ist; das axiale Loch mit dem radialen Loch verbunden ist; der Boden des Pufferentlüftungsringendes eng gegen den Boden der äußeren Hülle gedrückt ist.

8. Elektronische Zigarette, **dadurch gekennzeichnet, dass** die elektronische Zigarette einen oben montierten Zerstäuber für eine elektronische Zigarette nach einem der Ansprüche 1 bis 7 umfasst.

9. Verfahren zum Zusammensetzen des Zerstäubers nach einem der Ansprüche 1 bis 7, umfassend:
- ein getrenntes Zusammensetzen der Zerstäubungsvorrichtung und eines oberen Teils der Kopfabdeckungskomponente und
- eine Verbindung der zusammengesetzten Zerstäubungsvorrichtung mit einem unteren Ende des Lufteinlassrohrs von einem unteren Ende der Kopfabdeckungskomponente.

10. Verfahren zum Zusammensetzen eines Zerstäubers nach Anspruch 9, wobei das Zusammensetzen der Zerstäubungsvorrichtung umfasst:
- eine Installation einer Pufferentlüftungsringgruppe (10) in dem unteren Teil eines Zerstäubungskernkörpers (9) der Zerstäubungsvorrichtung;
- eine Installation eines becherförmigen Abschnitts (18) einer äußeren Hülle von dem unteren Teil des Zerstäubungskernkörpers (9);
- eine Installation eines Dichtungsrings auf einem Zerstäubungskernkörper (9);
- eine Installation eines zylindrischen Abschnitts (8) der äußeren Hülle von dem oberen Abschnitt des Zerstäubungskernkörpers; und
- eine Installation einer Pufferisolierringgruppe (16).

11. Verfahren zum Zusammensetzen eines Zerstäubers nach Anspruch 9 oder 10, wobei das Ansaugrohr als Referenz verwendet wird und wobei das obere Ende des Ansaugrohrs mit einer Verbindungsröhre vorgesehen ist, die zum Montieren eines Zigarettenhalters verwendet wird; wobei eine ringförmige Plattform des Verbindungsrohrs als eine Installationsverriegelungsplattform verwendet wird; wobei das Zusammensetzen des oberen Teils der Kopfabdeckungskomponente umfasst:
- eine Montage eines Lufteinlass-Einstellrings;
- eine Installation der Deckabdeckung der Lufteinlass-Ringkammer, der Seitenwand der Lufteinlass-Ringkammer und der Bodenabdeckung der Lufteinlass-Ringkammer; unter der Bodenabdeckung der Lufteinlass-Ringkammer, und
- eine Anordnung eines Isolierrings; unter dem Isolierring, und
- eine Anordnung eines Abdeckungskörpers eines Flüssigkeitsbeinhaltungsbechers.

12. Verfahren zum Zusammensetzen des Zerstäubers nach einem der Ansprüche 9 bis 10, ferner umfassend eine enge Verbindung des Abdeckungskörpers des Flüssigkeitsbeinhaltungsbechers mit dem Lufteinlassrohr der Kopfabdeckungskomponente.

13. Verfahren zum Zusammensetzen der elektronischen Zigarette nach Anspruch 8, umfassend:
- eine Einspritzung der E-Flüssigkeit in den Flüssigkeitsbeinhaltungsbecher, um eine Patrone zu bilden;
- eine Montage der Kopfabdeckungskomponente auf dem Flüssigkeitsbeinhaltungsbecher, der die E-Flüssigkeit enthält; und
- eine Montage des oben montierten Zerstäubers für eine elektronische Zigarette auf der elektronischen Zigarette.

## Revendications

1. Un atomiseur formant un ensemble supérieur d'une cigarette électronique, dans lequel ledit atomiseur comprend respectivement, de bas en haut:
- une coupelle de stockage de liquide (15) ayant une ouverture supérieure
- un élément de couvercle de tête (A) relié à l'ouverture supérieure de la coupelle de stockage de e-liquide (15);
dans lequel la coupelle de stockage de liquide (15) est reliée à l'élément de couvercle de tête (A) de manière fermée et filetée pour former un espace fermé dans la coupelle de stockage de liquide (15) destiné à contenir un e-liquide;
dans lequel l'élément de couvercle de tête (A) comprend un support de cigarette (1), un couvercle de réservoir de liquide, des électrodes de raccordement, un tuyau d'aspiration (5), un tuyau d'admission d'air (6) et un dispositif d'atomisation, et consiste en une pièce intégrée configurée pour être retirée dans son ensemble du réservoir de liquide (15), le tuyau d'aspiration (5), le tuyau d'admission d'air (6) et le dispositif d'atomisation se prolongeant vers le bas dans le réservoir de e-liquide (15);
dans lequel ledit couvercle de la coupelle de stockage de liquide présente, depuis le bas jusqu'au haut, une structure multicouche, les couches étant respectivement le corps du couvercle de la coupelle de stockage de liquide (14) servant également de première électrode (26),une bague isolant (13), une chambre annulaire d'admission d'air (3) ayant un couvercle supérieur et servant de seconde électrode (25), une bague régulatrice d'admission d'air (11), dans lequel le corps du couvercle de la coupelle de stockage de liquide est relié de manière annulaire, et électriquement, au tuyau d'admission d'air; dans lequel le couvercle de la chambre de la bague d'admission d'air et le tuyau d'aspiration sont reliés de manière annulaire et électriquement; la bague régulatrice d'admission d'air étant disposé dans la périphérie de la chambre de la bague d'admission d'air formant le joint tournant.

2. L'atomiseur formant un ensemble supérieur d'une cigarette électronique selon la revendication 1, **caractérisé en ce que** ledit dispositif d'atomisation comprend un corps de noyau d'atomisation (9) et une enveloppe extérieure, de préférence tous deux cylindriques.

3. L'atomiseur formant un ensemble supérieur d'une cigarette électronique selon la revendication 2, **caractérisé en ce que** le corps de noyau d'atomisation (9) est disposé de manière fixe dans l'enveloppe extérieure; l'extrémité supérieure du corps de noyau d'atomisation est reliée de manière annulaire et isolante à l'extrémité inférieure du tuyau d'aspiration, l'extrémité inférieure étant ouverte; **en ce que** l'extrémité supérieure de l'enveloppe extérieure est reliée de manière annulaire et électrique à l'extrémité inférieure du tuyau d'admission d'air (6), l'extrémité inférieure étant fermée; **en ce que** le corps de noyau d'atomisation est muni d'un dispositif électrique de chauffe et d'une entrée radiale de liquide (21); **en ce que** l'enveloppe extérieure est munie d'un trou d'entrée radial (17); **en ce que** la position de l'entrée de liquide de l'enveloppe extérieure correspond à la position de l'entrée de liquide du corps du noyau d'atomisation; **en ce que** le bord extérieur du corps du noyau d'atomisation entre en contact avec la paroi intérieure de l'enveloppe extérieure pour le raccordement, et une encoche de ventilation (22) est disposée entre le bord extérieur du corps du noyau d'atomisation et l'enveloppe extérieure; **en ce que** deux bornes du dispositif électrique de chauffe dudit corps du noyau d'atomisation sont connectées électriquement au corps du noyau d'atomisation et au tuyau d'aspiration respectivement.

4. L'atomiseur formant un ensemble supérieur d'une cigarette électronique selon la revendication 2 ou 3, **caractérisé en ce qu'**une encoche de ventilation (22) entre ledit corps de noyau d'atomisation et l'enveloppe extérieure est formée sur la paroi extérieure du corps de noyau d'atomisation ; la paroi extérieure est pourvue d'une encoche axiale qui est enfoncée vers l'axe; **en ce que** la partie encochée vient en contact avec l'enveloppe extérieure pour former l'encoche de ventilation (22).

5. L'atomiseur formant un ensemble supérieur d'une cigarette électronique selon l'une quelconque des revendications 2 à 4, **caractérisé en ce que** l'enveloppe extérieure dudit dispositif d'atomisation comprend une partie cylindrique (8) et une partie en forme de coupelle (18), **en ce que** la partie cylindrique (8) et la partie en forme de coupelle (18) sont interconnectées de manière fermée; **en ce que** ledit corps de noyau d'atomisation est disposé de manière fixe dans l'enveloppe extérieure du dispositif d'atomisation; **en ce que** l'extrémité supérieure cylindrique de l'enveloppe extérieure est reliée de manière annulaire au tuyau d'admission d'air (6) de manière fermée.

6. L'atomiseur formant un ensemble supérieur d'une cigarette électronique selon la revendication 5, **caractérisé en ce que** la partie cylindrique de ladite enveloppe extérieure est gainée sur la couche extérieure du corps du noyau d'atomisation; **en ce que** la partie en forme de coupelle et la partie inférieure du corps du noyau d'atomisation sont interconnectées de manière filetée; la partie cylindrique de ladite enveloppe extérieure et le tuyau d'admission d'air sont interconnectés de manière filetée.

7. L'atomiseur formant un ensemble supérieur d'une cigarette électronique selon l'une des revendications 2 à 6, **caractérisé en ce qu'**une bague isolante tampon (16) est disposée entre ledit corps de noyau d'atomisation et le tuyau d'aspiration; **en ce qu'**une bague de ventilation tampon (10) est disposée entre le corps de noyau d'atomisation et le fond de la partie en forme de coupelle de l'enveloppe extérieure; **en ce que** la section supérieure de ladite bague de ventilation tampon est pourvue d'un trou axial, et la section inférieure est pourvue d'un trou radial; **en ce que** le trou axial communique avec le trou radial; **en ce que** le fond de l'extrémité de la bague de ventilation tampon est pressé étroitement contre le fond de l'enveloppe extérieure.

8. Une cigarette électronique, **caractérisée en ce que** ladite cigarette électronique comprend un atomiseur formant un ensemble supérieur d'une cigarette électronique selon l'une des revendications 1 à 7.

9. Un procédé d'assemblage de l'atomiseur selon l'une des revendications 1 à 7, comprenant :
- un assemblage du dispositif d'atomisation et d'une partie supérieure de l'élément de couverture de la tête distincts l'un de l'autre, et
- un raccordement du dispositif d'atomisation assemblé à une extrémité inférieure du tuyau d'admission d'air depuis une extrémité inférieure de l'élément du couvercle de tête.

10. Le procédé d'assemblage d'un atomiseur selon la revendication 9, dans laquelle l'assemblage du dispositif d'atomisation comprend :
- une installation d'un groupe de bagues de ventilation tampon (10) dans la partie inférieure d'un corps de base d'atomisation (9) du dispositif d'atomisation;
- une installation, depuis la partie inférieure du corps du noyau d'atomisation (9), d'une partie extérieure en forme de coupelle (18);
- une installation, sur le corps du noyau d'atomisation (9), d'un joint d'étanchéité;
- une installation, à partir de la section supérieure du corps du noyau d'atomisation, d'une partie cylindrique de l'enveloppe extérieure (8)
- une installation d'un groupe de bagues de ventilation tampon (16).

11. Le procédé d'assemblage d'un atomiseur selon la revendication 9 ou 10, dans lequel le tuyau d'aspiration est utilisé comme référence et dans lequel l'extrémité supérieure du tuyau d'aspiration est munie d'un tube de raccordement utilisé pour monter un porte-cigarette; dans lequel une plate-forme annulaire du tube de raccordement est utilisée comme plate-forme de verrouillage de l'installation; l'assemblage de la partie supérieure de l'élément de couverture de la tête comprend :
- un montage d'une bague de réglage de l'admission d'air;
- une installation du couvercle supérieur de la chambre annulaire d'admission d'air, de la paroi latérale de la chambre annulaire d'admission d'air et du couvercle inférieur de la chambre annulaire d'admission d'air; sous le couvercle inférieur de la chambre annulaire d'admission d'air, et
- le positionnement d'une bague isolante; sous la bague isolante, et
- le positionnement d'un corps de couverture d'une coupelle de stockage de liquide.

12. Le procédé d'assemblage de l'atomiseur selon l'une des revendications 9 à 10, comprenant en outre un raccordement étanche du corps du couvercle de la coupelle de stockage de liquide avec le tuyau d'admission d'air du composant du couvercle de tête.

13. Le procédé d'assemblage de la cigarette électronique selon la revendication 8, comprenant :
- une injection du e-liquide dans la coupelle de stockage du liquide pour former une cartouche;
- un montage de l'élément du couvercle sur la coupelle de stockage du liquide contenant l'e-liquide
- un montage de l'atomiseur formant un ensemble supérieur de la cigarette électronique sur la cigarette électronique.
